# EUROPEAN PATENT SPECIFICATION

(11) **EP 0 485 608 B1**
(45) Date of publication and mention of the grant of the patent: **22.11.1995**
(21) Application number: 90912889.4
(22) Date of filing: 05.06.1990
(51) Int. Cl.: C12P 21/00, C12P 21/02, C12N 15/00

(54) **METHOD FOR OBTAINING POLYPEPTIDES IN A CELL-FREE TRANSLATION SYSTEM**
VERFAHREN ZUR HERSTELLUNG VON POLYPEPTIDEN IN EINEM ZELLFREIEN TRANSLATIONSSYSTEM
PROCEDE D'OBTENTION DE POLYPEPTIDES DANS UN SYSTEME DE TRANSLATION EXEMPT DE CELLULES

(30) Priority: 31.07.1989 SU 4717700
(43) Date of publication of application: 20.05.1992
(73) Proprietor: INSTITUT BELKA AKADEMII NAUK SSSR, Puschino 142292 (RU)
(72) Inventor: OVODOV, Sergei Jurievich, Puschino, 142292 (SU); BARANOV, Vladimir Ivanovich, Puschino, 142292 (SU); ALAKHOV, July Borisovich, Puschino, 142292 (SU); RYABOVA, Ljubov Anatolievna, Puschino, 142292 (SU)
(74) Representative: Goldin, Douglas Michael
(86) International application number: SU9000145
(87) International publication number: WO9102075

(56) References cited:
- WO-A-88/08453
- US-A- 4 382 028
- BIOPOLIMERI I KLETKA, vol. 4, no. 3, 1988, Naukova dumka, Kiew (UK); A.P. POTAPOW et al., pp. 133-138
- TRANSKRIPTSIA I TRANSLYATSIA. METODY, Pod. red. B. Keimsa & S. Sigginsa, 1987, Mir, Moskow (RU); pp. 216-217
- NUCLEIC ACIDS RESEARCH, vol. 9, no. 18, 1981, IRL Press Ltd., London (GB); J.M. PRATT, p. 4459
- SCIENCE, vol. 242, 25 November 1988; A.S. SPIRIN et al., pp. 1162-1164

## Description

This invention relates to molecular biology and bioengineering, and more particularly it relates to methods of preparing polypeptides in cell-free translation systems.

Said polypeptides are widely used in medicine as regulators of biological processes. Known in the prior art are, for example, polypeptides activating the immune systems, polypeptides neuromediators and transmitters, polypeptides regulators of salt metabolism, etc. Polypeptides are also used in agriculture as biological stimulants, e.g., growth hormones. Polypeptides are also used in bioelectronics, e.g., as rhodopsin films.

Known in the prior are two methods of preparing polypeptides: chemical synthesis and the genetic engineering method.

Chemical synthesis is widely used in industrial manufacture of polypeptides of comparatively small length (to 15 amino acid residues). Longer polypeptides cannot practically be prepared with this method because the yield of the end product decreases exponentially with increasing length of the polypeptide chain due to racemization of the amino-acid radicals, incomplete elongation of the polypeptide chain, and difficult selection of protective groups and their removal. All these factors interpose great difficulties in purification of the end product and drastically increase the cost of the polypeptides with their increasing length.

The genetic engineering method of preparative synthesis of polypeptides also has certain limitations in practical use. These are connected with difficult isolation of the expression products by the transformed cells, the fatal effect that some end products produce on the product cell elimination of transformed plasmids from the cell, and the proteolytic degradation of the expression product of a foreign gene.

The described methods of preparing polypeptides cannot practically be used to obtain polypeptides 15-70 amino acid residues long. However, this is just the length of many biologically active polypeptides having large application in medicine, agriculture, bioelectronics.

There is another method of preparative synthesis of polypeptides based on the use of a continuous cell-free translation system.

Known in the prior art is, for example, a method of calcitonin synthesis in a continuous cell-free translation system from wheat embryo lysate (Science, 1988, v. 242, A.S. Spirin, V.I. Baranov, L.A. Ryabova, S.Yu. Ovodov, Yu.B. Alakhov, A Continuous Cell-Free Translation System Capable of Producing Poly-peptides in High Yield, pp. 1162-1164).

EP-A-0 312 617 and SCIENCE, vol. 242, 25/11/88, pp.1162-64 describe a continuous cell-free translation system. Said system is placed in a ultrafiltration cell containing a semipermeable membrane through which the translation products are collected.

1 ml of the cell-free system contains 0.5 ml of wheat embryo lysate, 0.1 nmol calcitonin mRNA, 64 µg of creatine phosphokinase, 10 µg of ribonuclease inhibitor from human placenta, 0.1 µg of each protease inhibitor (aprotinin, pepstatin, leupeptin) in buffer: 40 mM HEPES, pH 7.6, 75 mM K⁺ acetate, 1.9 mM Mg²⁺ acetate, 0.25 mM spermidine, 6 mM dithiothreitol, 1.5% glycerol, 2 mM ATP, 50 µM GTP, 8 mM creatine phosphate, 25 µm [³H]leucine (activity 50 Ci/mmol) and 25 µm of each of the remaining 19 amino acids.

The cell-free system is placed in a diafiltration cell (Amicon) and polypeptide synthesis is performed at 25°C. The translation products including the end product and decomposition products (AMP, GDP, pyrophoshate and inorganic phosphate) are collected through a semipermeable membrane, substrates (ATP, GTP and amino acids) are simultaneously supplied during 20 hrs. As a result, operation of the cell-free translation system yields 10 nmol of calcitonin, which makes 100 pmol of polypeptide per 1 pmol of mRNA.

This method of preparing polypeptides is the most efficient among the known methods. Since the said method uses a cell-free translation system, synthesis of practically any polypeptides can be performed.

However, in this method the removal of the end polypeptide and low molecular weight reaction products (GDP, AMP, pyrophoshate, inorganic phosphate) requires the use of expensive ultrafiltration equipment. The very principle of ultrafiltration considerably limits the possibilities of the method owing to aggregation of the end product on the membrane and the necessity of performing synthesis in rather large volumes of the reaction mixture leads to inactivation of enzymes of the protein-synthesizing system under the influence of different physical and chemical factors. All these circumstances limit the period of the system operation and hinder its use at industrial scale.

An object of the present invention is to provide a method of preparing polypeptides in a cell-free translation system which would prolong the period of the translation system work and would allow its reproduction at a large scale, at the same time excluding the use of the expensive ultrafiltration equipment.

This object is accomplished by provision of the method of preparing polypeptides in a cell-free translation system on ribosomes containing template RNA, substrates in the form of ATP, GTP and amino acids, with the formation of translation products including the end polypeptide, AMP, GDP, pyrophosphate and inorganic phosphate which are removed from the translation system while the substrates are consumed and at the same time substrates in the form of ATP, GTP and amino acids are introduced into the system to maintain their initial concentration, in which, according to the invention, a system of translation on ribosomes is used including a template RNA and substrates in the form of ATP, GTP, amino acids immobilized in microcapsules whose envelope represents polyelectrolye complexes.

Procaryotic and eucaryotic cell-free translation systems containing both endogenous and exogenous natural or artificial mRNAs are used as cell-free translation systems according to the invention. This method uses immobilization systems based on negatively charged polysaccharides and positively charged polymers forming polyelectrolyte complexes with polysaccharides and allowing formation of microcapsules whose envelope is practically impermeable for cell-free system proteins, but does not retain the reaction products (the end polypeptide, AMP, GDP, pyrophosphate, inorganic phosphate) and the substrates (ATP, GTP, amino acids). Such complexes can be formed, e.g., by Na alginate and poly-L-lysine, Na alginate and chitosan, pectin and polyamine, pectin and chitosan.

The present invention provides a method for preparing a polypeptide in a cell-free translation system, comprising
preparing a solution containing the cell-free protein translation system including
a) ribosomes,
b) substrates ATP, GTP and amino acids,
c) an mRNA encoding the selected peptide, immobilizing the cell-free protein translation system in a microcapsule having a polyelectrolyte complex envelope which is practically impermeable to proteins of the cell-free system but which does not retain the substrates or translation products including end polypeptide, AMP, GDP, pyrophosphate and inorganic phosphate, and
   during translation, introducing into the system said substrates to maintain an initial concentration of the substrates.

The invention also provides a microcapsule composition for use in the said method.

The proposed method of synthesis of polypeptides in cell-free translation system has no disadvantages that are inherent in the ultrafiltration method, is easily reproducible at large scale and can be used to produce polypeptides of any length and amino acid sequence.

The proposed method ensures the synthesis of polypeptides in cell-free translation system at a constant rate during 100 hours and more. The amount of the end peptide is 400-500 polypeptide copies per mRNA copy.

The invention will further be described with reference to the appended drawings in which:

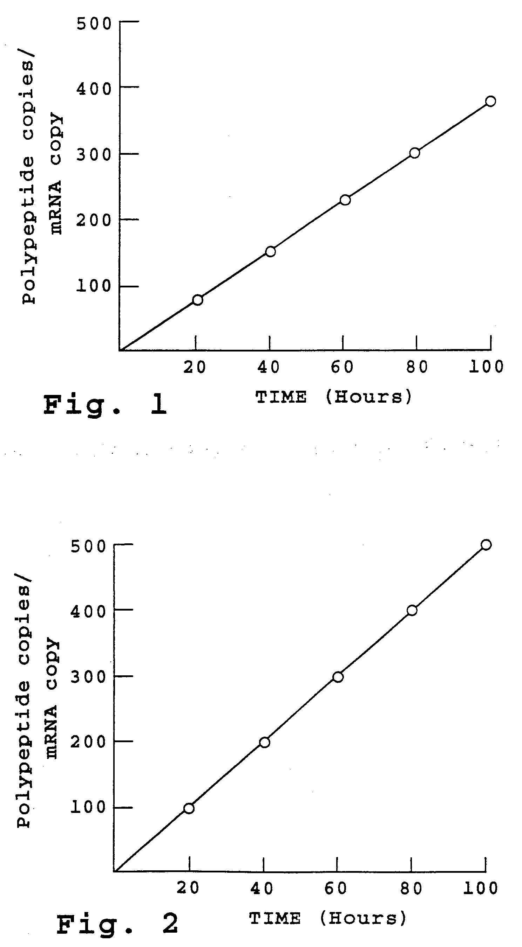

Figs. 1, 2 present graphic dependence of the quantity of the synthesized polypeptide per mRNA unit vs. the time of synthesis.

The method of preparing polypeptides in a cell-free translation system is technologically simple and can be realized as follows.

A cell-free translation system of procaryotic or eucaryotic organisms, containing ribosomes, translation factors, tRNA, mRNA, reaction substrates including ATP, GTP and amino acids, is prepared by the known methods.

The cell-free translation system is mixed with a solution of negatively charped polysaccharide and is introduced through a drop-forming device into a solution of positively charged polymer. The microcapsules formed are washed with a buffer solution to remove unreacted polymer and are placed onto a column, through which a buffer solution containing substrates necessary for translation is passed.

During the synthesis the reaction products are removed from microcapsules through the semipermeable membrane. Simultaneously, the substrates in the form of ATP, GTP and amino acids are supplied to the system.

For a further isolation and purification of the end product, the solution passed through the column with microcapsules is supplied into the column packed with immunoaffinity sorbent, where selective absorption of the end product takes place. After termination of the synthesis, the end product is eluted from the column and desalted.

To understand better the present invention, the following examples are given hereinbelow. Example 1.

Calcitonin synthesis in a cell-free eucaryotic translation system from wheat embryos immobilized into microcapsules form Na alginate and polylysine proceeds in the following way.

Solution (A) consisted of 35 mM HEPES pH 7.6, 2.1 mM Mg(OAc)₂, 70 mM KoAc, 1 mM ATP, 25 µM GTP, 250 µM spermidine, 25 µM [³H]leucine with specific radioactivity of 24 Ci/mmol. 25 µM of each of the 19 remaining amino acids, 8 mM creatine phosphate, 2.5 ml of the incubation mixture prepared from solution (A) contained 80 absorbance units A₂₆₀ lysate from wheat embryo (S30), 80 pmol of calcitonin mRNA, 150 activity units of human placenta ribonuclease inhibitor. The prepared incubation mixture was immobilized in alginate-poly L-lysine capsules according to the following methods.

2.5 ml of the incubation mixture were mixed with 2.5 ml of 2% Na alginate. The obtained mixture was introduced through a drop-forming device into a 1.2% solution of Ca chloride. The obtained maicrogranules were washed thrice with solution (B) containing 120 mM KoAc and 2 mM Md (oAc)₂. Solution B was removed and the granules were flooded with a 0.025% poly-L-lysine solution (MW-50000) and incubated for six minutes. After removal of the poly-L-lysine solution the granules were washed twice with buffer B, placed in 0.6% polyimine P with buffer (A) at the rate of 3 ml/hr. The eluate was collected and analyzed for the presence of synthesized product.

Fig. 1 represents the dependence of the amount of the obtained calcitonin per unit of template RNA vs. the time of synthesis. The abscissa represents the time of synthesis in hours and the ordinate-the amount of the product formed in pmoles per unit of template RNA in pmoles. As a result 32000 pmoles were obtained in 100 hours.

### Example 2.

Synthesis of phage MS2 coat protein on phage MS2 mRNA template in a cell-free procaryotic translation system with the example of incubation mixture polymerized in alginate gel was carried out as follows.

1 ml of the reaction mixture containing 0.6 nmol of 70S ribosomes, 1 mg of S100 fraction, 0.6 mg of tRNA, 0.06 nmol of mRNA of phage MS2 coat protein, 5 µg of pyruvate kinase, 2-10 µg of human placenta ribonuclease inhibitor, 0.1 µg of each protease inhibitor (aprotinin, leupeptin, chymostatin) in buffer A: 20 mM Tris-HCl, pH 7.5, 10 mM MgCl₂, 100 mM NH₄Cl, 1 mM ATP, 0.2 mM GTP, 5 mM phosphoenolpyruvate, 25 µm [³H]leucine with specific radioactivity of 52 Ci/mmol and 25 µm each of the 19 remaining amino acids.

Immobilization of the incubation mixture in microcapsules was performed as described in Example 1. The obtained microcapsules were placed onto the column and washed with buffer A. Synthesis in the column was carried out at 37°C. The end product and the decomposition products were collected by withdrawing the feeding mixture passed through the column with a simultaneous supply of buffer A to the column during 100 hours.

Fig. 2 represents the dependence of the amount of the obtained coat protein per unit of template RNA vs. the time of synthesis. As a result, 30000 pmoles of phage MS-2 coat protein were synthesized in 100 hours of the work of the cell-free translation system.

The polypeptides prepared according to the invention can be used in medicine, agriculture, and bioelectronics.

## Claims

1. A method for preparing a polypeptide in a cell-free translation system, comprising
preparing a solution containing the cell-free protein translation system including
a) ribosomes,
b) substrates ATP, GTP and amino acids,
c) an mRNA encoding the selected peptide,
immobilizing the cell-free protein translation system in a microcapsule having a polyelectrolyte complex envelope which is practically impermeable to proteins of the cell-free system but which does not retain the substrates or translation products including end polypeptide, AMP, GDP, pyrophosphate and inorganic phosphate, and
during translation, introducing into the system said substrates to maintain an initial concentration of the substrates.

2. The method of claim 1, wherein the polyelectrolyte complex envelope is formed from a negatively charged polysaccharide and a positively charged polymer.

3. The method of claim 2, wherein the polysaccharide is selected from the group consisting of Na-alginate and pectin, and the polymer is selected from the group consisting of poly-L-lysine, chitosan, and polyamine.

4. A microcapsule composition for use in producing a selected peptide, comprising
a microcapsule having a polyelectrolyte complex envelope,
contained within the microcapsule envelope, the components of a cell-free protein translation system including
a) ribosomes,
b) substrates ATP, GTP and amino acids,
c) transfer RNA, and
d) an mRNA encoding the selected peptide,
wherein said microcapsule envelope is practically impermeable to proteins of the cell-free system and said envelope does not retain the substrates or translation products including end polypeptide, AMP, GDP, pyrophosphate and inorganic phosphate.

5. The composition of claim 4, wherein the microcapsule envelope is formed from a negatively charged polysaccharide and positively charged polymer.

6. The composition of claim 5, wherein the polysaccharide is selected from the group consisting of Na-alginate and pectin and the polymer is selected from the group consisting of polylysine, chitosan, and polyamine.

## Patentansprüche

1. Verfahren zur Herstellung eines Polypeptids in einem zellfreien Translationssystem, umfassend
das Herstellen einer das zellfreie Protein-Translationssystem enthaltenden Lösung, einschließend
a) Ribosomen,
b) die Substrate ATP, GTP und Aminosäuren, und
c) eine das gewählte Peptid kodierende mRNA,
das Immobilisieren des zellfreien Protein-Translationssystems in einer Mikrokapsel mit einer Polyelektrolyt-Komplexhülle, welche praktisch für Proteine des zellfreien Systems undurchlässig ist, welche jedoch nicht die Substrate oder Translationsprodukte, einschließlich des Endpolypeptids, AMP, GDP, Pyrophosphats und anorganischen Phosphats, zurückhält, und
das Einführen der Substrate in das System während der Translation, um eine anfängliche Konzentration der Substrate zu erhalten.

2. Verfahren nach Anspruch 1, worin die Polyelektrolyt-Komplexhülle aus einem negativ geladenen Polysaccharid und einem positiv geladenen Polymer gebildet wird.

3. Verfahren nach Anspruch 2, worin das Polysaccharid aus der aus Na-Alginat und Pektin bestehenden Gruppe gewählt wird, und das Polymer aus der aus Poly-L-lysin, Chitosan und Polyamin bestehende Gruppe gewählt wird.

4. Mikrokapselzusammensetzung zur Verwendung bei der Herstellung eines gewählten Peptids, umfassend
eine Mikrokapsel mit einer Polyelektrolyt-Komplexhülle,
die innerhalb der Mikrokapselhülle vorliegenden Komponenten eines zellfreien Protein-Translationssystems, einschließend
a) Ribosomen,
b) die Substrate ATP, GTP und Aminosäuren,
c) Transfer-RNA, und
d) eine das gewählte Peptid kodierende mRNA,
worin die Mikrokapselhülle für Proteine des zellfreien Systems praktisch undurchlässig ist und die Hülle die Substrate oder Translationsprodukte, einschließlich des Endpolypeptids, AMP, GDP, Pyrophosphats und anorganischen Phosphats, nicht zurückhält.

5. Zusammensetzung nach Anspruch 4, worin die Mikrokapselhülle aus einem negativ geladenen Polysaccharid und einem positiv geladenen Polymer gebildet ist.

6. Zusammensetzung nach Anspruch 5, worin das Polysaccharid aus der aus Na-Alginat und Pektin bestehenden Gruppe und das Polymer aus der aus Polylysin, Chitosan und Polyamin bestehenden Gruppe gewählt ist.

## Revendications

1. Méthode pour préparer un polypeptide dans un système acellulaire de traduction, comprenant les étapes consistant à
préparer une solution contenant le système acellulaire de traduction en protéines comprenant
a) des ribosomes,
b) des substrats ATP, GTP et acides aminés, et
c) un ARNm codant pour le peptide choisi,
immobiliser le système acellulaire de traduction en protéines dans une microcapsule ayant une enveloppe à base d'un complexe de polyélectrolyte qui est pratiquement imperméable aux protéines du système acellulaire mais qui ne retient pas les substrats ou les produits de traduction, y compris les polypeptides final, AMP, GDP, pyrophosphate et phosphate inorganique, et
introduire dans le système, durant la traduction, lesdits substrats ou maintenir une concentration initiale des substrats.

2. Méthode selon la revendication 1, dans laquelle l'enveloppe a base d'un complexe polyélectrolyte est formée à partir d'un polysaccharide chargé négativement et d'un polymère chargé positivement.

3. Méthode selon la revendication 2, dans laquelle le polysaccharide est choisi dans le groupe constitué par le Na-Alginate et la pectine, et le polymère est choisi dans le groupe consistué par la poly-L-lusine, le chitosane, et la polyamine.

4. Composition à base de microcapsule pour usage dans la production d'un peptide choisi, comprenant
une microcapsule ayant une enveloppe à base d'un complexe polyélectrolyte,
les composants d'un système acellulaire de traduction en protéines, contenus à l'intérieur de l'enveloppe de la microcapsule, comprenant
a) des ribosomes,
b) des substrats ATP, GTP et acides aminés, et
c) un ARN de transfert, et
d) un ARNm codant pour le peptide chois,
où ladite enveloppe de la microcapsule est pratiquement imperméable aux protéines du système acellulaire et où ladite enveloppe ne retient pas les substrats ou les produits de traduction y compris les polypeptides final, AMP, GDP, pyrophosphate et phosphate inorganique.

5. Composition selon la revendication 4, dans laquelle l'enveloppe de la microcapsule est formée à partir d'un polysaccharide chargé négativement et d'un polymère chargé positivement.

6. Composition selon la revendication 5, dans laquelle le polysaccharide est choisi dans le groupe consistué par le Na-alginate et la pectine et le polymère est choisi dans le groupe consistué par la polylysine, le chitosane et la polyamine.
